(19) Europäisches Patentamt
European Patent Office
Office européen des brevets

(11) EP 4 790 385 A1

(12) **EUROPEAN PATENT APPLICATION**
published in accordance with Art. 153(4) EPC

(43) Date of publication:
**12.08.2026 Bulletin 2026/33**

(21) Application number: **24885838.3**

(22) Date of filing: **31.10.2024**

(51) International Patent Classification (IPC):
***G01N 21/27*** *(2006.01)* ***G01N 33/08*** *(2006.01)*

(52) Cooperative Patent Classification (CPC):
**G01N 21/27; G01N 21/359; G01N 33/08**

(86) International application number:
**PCT/JP2024/038918**

(87) International publication number:
**WO 2025/095071 (08.05.2025 Gazette 2025/19)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **01.11.2023 JP 2023187783**

(71) Applicants:
- **Kewpie Corporation**
  **Tokyo 150-0002 (JP)**
- **Hiroshima University**
  **Higashihiroshima-shi**
  **Hiroshima 739-8511 (JP)**

(72) Inventors:
- **YAMAMOTO, Hiroshi**
  **Chofu-shi, Tokyo 182-0002 (JP)**
- **SAWADA, Mami**
  **Chofu-shi, Tokyo 182-0002 (JP)**
- **KODAMA, Daisuke**
  **Chofu-shi, Tokyo 182-0002 (JP)**
- **HORIUCHI, Hiroyuki**
  **Higashihiroshima-shi, Hiroshima 739-8511 (JP)**
- **EZAKI, Ryo**
  **Higashihiroshima-shi, Hiroshima 739-8511 (JP)**

(74) Representative: **Winter, Brandl - Partnerschaft mbB**
**Alois-Steinecker-Straße 22**
**85354 Freising (DE)**

# (54) METHOD FOR SELECTING OVOMUCOID-FREE SHELLED CHICKEN EGGS

(57) The present invention relates to a method for selecting shelled chicken eggs, the method being a selecting method for ovomucoid-free shelled chicken eggs, including: a measurement step of irradiating a shelled chicken egg with light including light having a specific wavelength to measure an absorption amount of the light having the specific wavelength; a calculation step of calculating a discriminant value by substituting the absorption amount into a conversion formula; and a selection step of selecting an ovomucoid-free shelled chicken egg on the basis of the discriminant value, in which the conversion formula is a formula derived from a result of measuring the absorption amount of the light having the specific wavelength for an ovomucoid-containing shelled chicken egg and an ovomucoid-free shelled chicken egg.

EP 4 790 385 A1

## Description

### Technical Field

The present invention relates to a method for selecting ovomucoid-free shelled chicken eggs.

### Background Art

As a method for measuring components in chicken eggs, various technical means have been proposed. For example, Patent Literature 1 discloses a method for determining a shelled egg, the method including a step of irradiating a shelled egg with light, a step of receiving transmitted light of the shelled egg, a step of acquiring a spectrum of the transmitted light, a step of performing multivariate analysis on the spectrum, and a step of determining the shelled egg on the basis of the spectrum. Furthermore, Patent Literature 2 discloses that by irradiating a chicken egg with near infrared rays in a specific wavelength region and detecting transmitted light, the cholesterol content in the chicken egg can be quantified with high accuracy without cracking the egg.

### Citation List

### Patent Literature

Patent Literature 1: Japanese Unexamined Patent Publication No. 2017-49177
Patent Literature 2: Japanese Patent No. 5959061

### Summary of Invention

### Technical Problem

As one of substances to be allergens in chicken eggs, for example, a protein called ovomucoid contained in the egg white is known. Since ovomucoid is stable to heat and digestive enzymes, its allergen property is hardly lost even when a heat treatment and processing using a digestive enzyme are performed. For this reason, studies have been made on making a chicken egg not containing ovomucoid.

On the other hand, the ovomucoid in a chicken egg is generally measured by, for example, a liquid chromatography method, an ELISA method, or the like using the egg white obtained by cracking the egg. However, such a measurement method is not suitable commercially because the procedure is complicated and the egg is cracked. Therefore, it is desired to develop a method for nondestructively and conveniently selecting ovomucoid-free shelled chicken eggs.

An object of the present invention is to provide a method for nondestructively and conveniently selecting ovomucoid-free shelled chicken eggs.

### Solution to Problem

The present inventors have found that it is possible to determine an ovomucoid-free shelled chicken egg on the basis of a discriminant value by irradiating a shelled chicken egg with light including light having a specific wavelength, measuring an absorption amount of the light having the specific wavelength, and then calculating the discriminant value from the absorption amount by using a predetermined conversion formula, thereby completing the present invention.

That is, the present invention relates to, for example, the following inventions.

[1] A method for selecting shelled chicken eggs, the method being a selecting method for ovomucoid-free shelled chicken eggs, including:

a measurement step of irradiating a shelled chicken egg with light including light having a specific wavelength to measure an absorption amount of the light having the specific wavelength;
a calculation step of calculating a discriminant value by substituting the absorption amount into a conversion formula; and
a selection step of selecting an ovomucoid-free shelled chicken egg on the basis of the discriminant value, in which

the conversion formula is a formula derived from a result of measuring the absorption amount of the light having the specific wavelength for an ovomucoid-containing shelled chicken egg and an ovomucoid-free shelled chicken egg.

[2] The selecting method according to [1], in which the light having the specific wavelength is light existing within a wavelength range of 310 nm or more and 2500 nm or less.
[3] The selecting method according to [1] or [2], in which the conversion formula is a formula derived by partial least squares-discriminant analysis.
[4] The selecting method according to any one of [1] to [3], in which the conversion formula is a formula derived by partial least squares-discriminant analysis with a discriminant value of the ovomucoid-containing shelled chicken egg as 1 and a discriminant value of the ovomucoid-free shelled chicken egg as 0,

in the selection step, in a case where the discriminant value calculated in the calculation step is less than a threshold, the egg is selected as an ovomucoid-free shelled chicken egg, and
the threshold is within a range of more than 0 and 0.6 or less.

[5] A method for producing ovomucoid-free shelled chicken eggs, the producing method including:
a step of selecting an ovomucoid-free shelled chicken egg by performing the selecting method according to any one of [1] to [4].

## Advantageous Effects of Invention

According to the present invention, it is possible to provide a method for nondestructively and conveniently selecting ovomucoid-free shelled chicken eggs.

## Description of Embodiments

Hereinafter, embodiments for carrying out the present invention will be described in detail. However, the present invention is not limited to the following embodiments.

(Method for Selecting Ovomucoid-Free Shelled Chicken Egg)

The present invention is characterized by providing a method for selecting ovomucoid-free shelled chicken eggs, including: a measurement step of irradiating a shelled chicken egg with light including light having a specific wavelength to measure an absorption amount of the light having the specific wavelength; a calculation step of calculating a discriminant value by substituting the absorption amount into a conversion formula; and a selection step of selecting an ovomucoid-free shelled chicken egg on the basis of the discriminant value, in which the conversion formula is a formula derived from a result of measuring the absorption amount of the light having the specific wavelength for an ovomucoid-containing shelled chicken egg and an ovomucoid-free shelled chicken egg.

(Measurement Step)

The method for selecting ovomucoid-free shelled chicken eggs according to the present embodiment includes a measurement step of irradiating a shelled chicken egg with light including light having a specific wavelength to measure an absorption amount of the light having the specific wavelength.

(Shelled Chicken Egg)

In the present specification, the "shelled chicken egg" is an egg in a non-destructive state including all of an eggshell, an egg yolk, and an egg white. As long as the shelled chicken egg includes all of an eggshell, an egg yolk, and an egg white, the shelled chicken egg may be one in which the eggshell has cracks, or the like. As the shelled chicken egg, a shelled chicken egg used as a food material or the like can be used. There is no particular limitation on the chicken species of the laying hens and/or the time of egg collection in shelled chicken eggs. The eggshell of the shelled chicken egg may be a white eggshell or a colored eggshell such as a brown or reddish brown eggshell.

(Ovomucoid-Free Shelled Chicken Egg)

In the present specification, the "ovomucoid-free shelled chicken egg" is a shelled chicken egg not containing

ovomucoid, and is as described above for the shelled chicken egg except that ovomucoid is not contained. Ovomucoid is a protein contained in the egg white, and is one of substances that become allergens of chicken eggs. Furthermore, ovomucoid is water-soluble and has extremely high physicochemical stability. Therefore, even when a heat treatment and processing using a digestive enzyme are performed, the allergen property is hardly lost.

In the present specification, "not containing ovomucoid" means that the content of ovomucoid in the egg white is less than 1 μg/mL, and preferably 0 μg/mL based on the total amount of the egg white.

(Use Application of Ovomucoid-Free Shelled Chicken Egg)

Examples of use applications of the ovomucoid-free shelled chicken egg include use applications for boiled egg, making confectionery, cooking, breadmaking, frozen dessert, water mixing, ham, noodle, and the like. The use application for boiled egg means a use application as a raw material of boiled egg. The use application for making confectionery means a use application as a raw material for producing confectionery. The ovomucoid-free shelled chicken egg for making confectionery can be used as, for example, a raw material for sponge cake or the like. The use application for cooking means a use application as a raw material for producing food and drink (provided that confectionery is excluded). The ovomucoid-free shelled chicken egg for cooking can be used as, for example, a raw material for iri-tamago (Japanese scrambled eggs), scrambled eggs, egg soup, fried egg, omelet, or rolled omelet. The method for selecting ovomucoid-free shelled chicken eggs according to the present embodiment can also be used as a method for selecting ovomucoid-free shelled chicken eggs for boiled eggs, for making confectionery, or for cooking. Furthermore, in particular, since ovomucoid is a protein having an allergen property, the ovomucoid-free shelled chicken egg can also be used for the development of low-allergen foods. The low-allergen foods mean foods which hardly cause an allergy.

The ovomucoid-free shelled chicken egg may be distributed in the form of a shelled chicken egg, may be distributed in the form containing the egg white and the egg yolk by cracking the egg, or may be distributed in the form of the egg white or the form of the egg yolk after further separating the egg white and the egg yolk by cracking the egg.

(Method of Measuring Absorption Amount of Light Having Specific Wavelength)

The absorption amount of the light having the specific wavelength (hereinafter, also referred to as "absorption amount") is measured using a spectroscopic method in which a shelled chicken egg is irradiated with light including light having a specific wavelength and transmitted light or reflected light is detected. That is, in the measurement step, the absorption amount is measured by irradiating a shelled chicken egg with light including light having a specific wavelength and detecting transmitted light or reflected light. The absorption amount to be measured may be an absolute value or a relative value (for example, absorbance or the like). Furthermore, the absorption amount to be measured may be the absorption amount of light having a single wavelength, or may be the absorption amount of light having multiple wavelengths. In a case where the absorption amount to be measured is the absorption amount of light having multiple wavelengths, the light may be light having continuous multiple wavelengths, or may be light having discontinuous multiple wavelengths. In a case where the absorption amount of light having multiple wavelengths is measured, for example, an absorption spectrum including the absorption amount of light having a specific wavelength may be measured.

In the method for selecting ovomucoid-free shelled chicken eggs according to the present embodiment, it is preferable to measure the absorption amount by detecting transmitted light or reflected light. The absorption amount can be measured by, for example, a visible near infrared spectrophotometer, a near infrared spectrophotometer, or the like. As the visible near infrared spectrophotometer, for example, Handy Lambda II NIR enh manufactured by Spectra Co-op., or the like can be used. As the near infrared spectrophotometer, for example, a near infrared spectrometer Matrix-F manufactured by Bruker Optics GmbH & Co. KG, or the like can be used. Analysis of the obtained absorption amount data can be performed using commercially available software. Examples of the commercially available software include The Unscrambler X (manufactured by CAMO Software), and OPUS (manufactured by Bruker Optics GmbH & Co. KG).

(Light Having Specific Wavelength)

The light having the specific wavelength in the measurement step may be, for example, light existing within a wavelength range of 310 nm or more and 2500 nm or less, 310 nm or more and 2000 nm or less, 310 nm or more and 1500 nm or less, 310 nm or more and 1100 nm or less, 600 nm or more and 2500 nm or less, 800 nm or more and 2500 nm or less, or 1100 nm or more and 2500 nm or less. As described above, the light having the specific wavelength may be light existing within the range of the wavelength region, may be light having a single wavelength in the wavelength region, or may be light having a plurality of wavelengths in the wavelength region. The absorption amount of the light having the wavelength within this wavelength range into the shelled chicken egg changes between the ovomucoid-free shelled chicken egg and the ovomucoid-containing shelled chicken egg, and thus it is possible to select the ovomucoid-free shelled chicken egg on the basis of the absorption amount.

(Wavelength of Light Applied to Shelled Chicken Egg)

The light with which the shelled chicken egg is irradiated in the measurement step (hereinafter, also referred to as "irradiation light") is not particularly limited as long as the light is light including the light having the specific wavelength. That is, the irradiation light may include light having a wavelength other than the light having the specific wavelength, or may be the light having the specific wavelength. The absorption amount is measured by irradiating a shelled chicken egg with irradiation light. The irradiation light may be, for example, light in a wavelength region of 100 nm or more and 3000 nm or less, or may be light in a wavelength region of 150 nm or more and 2500 nm or less, 200 nm or more and 2000 nm or less, 300 nm or more and 1500 nm or less, 310 nm or more and 1100 nm or less, 500 nm or more and 3000 nm or less, 600 nm or more and 3000 nm or less, 700 nm or more and 2800 nm or less, 750 nm or more and 2600 nm or less, or 800 nm or more and 2500 nm or less.

(Arrangement Conditions of Shelled Chicken Eggs)

In the measurement step, for example, the shelled chicken egg may be arranged such that a major axis connecting a rounded end and a pointed end of the shelled chicken egg is parallel or substantially parallel to the horizontal plane, or may be arranged such that the major axis is perpendicular or substantially perpendicular to the horizontal plane. In a case where the major axis is disposed perpendicular or substantially perpendicular to the horizontal plane, the shelled chicken egg may be arranged such that the rounded end faces downward. In the measurement step, the shelled chicken egg may be fixed so as to be in the above arrangement.

(Irradiation Conditions of Light Applied to Shelled Chicken Egg)

In the measurement step, the irradiation light can be applied to the shelled chicken egg from various directions. The irradiation with the irradiation light may be performed in a state where the shelled chicken egg is fixed such that the shelled chicken egg is in the above arrangement, or may be performed in a non-fixed state. The irradiation light may be applied, for example, in a direction intersecting a major axis connecting a rounded end and a pointed end of the shelled chicken egg, or may be applied along the major axis. The absorption amount may be measured, for example, by applying irradiation in a direction intersecting a major axis connecting a rounded end and a pointed end of the shelled chicken egg in a state where the major axis is disposed to be parallel or substantially parallel to the horizontal plane, and detecting transmitted light or reflected light. Furthermore, the absorption amount may be measured, for example, by applying irradiation light in a direction intersecting a major axis connecting a rounded end and a pointed end of the shelled chicken egg in a state where the rounded end of the shelled chicken egg faces downward and the major axis is disposed to be perpendicular or substantially perpendicular to the horizontal plane, and detecting transmitted light or reflected light.

(Detection of Light Having Specific Wavelength)

In a case where the irradiation light is applied and transmitted light is detected, from the viewpoint of enabling more accurate measurement, it is preferable to detect the transmitted light obtained by irradiating a portion through which the light does not pass the egg yolk with the irradiation light. In a case where the irradiation light is applied and reflected light is detected, reflected light derived from an egg white site in the shelled chicken egg can also be detected.

(Calculation Step)

The method for selecting ovomucoid-free shelled chicken eggs includes a calculation step of calculating a discriminant value by substituting the absorption amount into a conversion formula.

(Conversion Formula)

In the calculation step, the conversion formula is used for calculating a discriminant value from the absorption amount measured in the measurement step. The conversion formula is a formula derived from the result of measuring the absorption amount for an ovomucoid-containing shelled chicken egg and an ovomucoid-free shelled chicken egg. More specifically, it is a relational expression between the absorption amount and the discriminant value, the relational expression being derived with the measured absorption amount as an explanatory variable and the discriminant value as a dependent variable. The conversion formula may be, for example, a formula derived by multivariate analysis, or may be a formula derived by partial least squares-discriminant analysis (PLS-DA), logistic regression analysis, or the like, and is preferably a formula derived by partial least squares-discriminant analysis. The discriminant value is a numerical value set by associating a predetermined numerical value with each of the ovomucoid-containing shelled chicken egg and the

ovomucoid-free shelled chicken egg, and is a numerical value for discriminating the ovomucoid-free shelled chicken egg. For example, these chicken eggs and numerical values may be associated with each other by setting the discriminant value of the ovomucoid-containing shelled chicken egg to 1 and the discriminant value of the ovomucoid-free shelled chicken egg to 0. In this case, the conversion formula may be a formula derived by partial least squares-discriminant analysis from the result of measuring the absorption amount with the discriminant value of the ovomucoid-containing shelled chicken egg as 1 and the discriminant value of the ovomucoid-free shelled chicken egg as 0.

(Multivariate Analysis)

The multivariate analysis is, for example, a statistical technique used for classifying a subject by simultaneously analyzing a plurality of pieces of data (variable, variate) obtained from the subject, analyzing a correlation between the plurality of pieces of data, and comprehensively summarizing the plurality of pieces of data. The multivariate analysis may optionally be performed in combination with a spectral filtering method, for example, orthogonal signal correction (OSC) to remove interfering components. Many analysis tools are commercially available as software, and any analysis tool is available. Such a commercially available tool is generally provided with an operation manual so that multivariate analysis can be performed without knowledge of difficult mathematics and statistics. The multivariate analysis may be performed using a part or all of the obtained analysis data.

(Partial Least Squares-Discriminant Analysis (PLS-DA))

PLS-DA is a method of applying partial least squares (PLS) to discriminant analysis (DA) to construct a model that discriminates between two groups. In the method for selecting ovomucoid-free shelled chicken eggs according to the present embodiment, a model (conversion formula) for discriminating between an ovomucoid-containing shelled chicken egg and an ovomucoid-free shelled chicken egg from the measured absorption amount is constructed.

(Ovomucoid-Containing Shelled Chicken Egg in Calculation Step)

The ovomucoid-containing shelled chicken egg in the calculation step is used to derive the above-described conversion formula. The ovomucoid-containing shelled chicken egg is as described in the above-described shelled chicken egg except that the shelled chicken egg contains ovomucoid. Examples of the ovomucoid-containing shelled chicken egg in the calculation step include shelled chicken eggs obtained from laying hens of wild-type chicken species. The ovomucoid-containing shelled chicken egg in the calculation step may be known to be an ovomucoid-containing shelled chicken egg when the absorption amount of the light having the specific wavelength is measured, or may be found to be an ovomucoid-containing shelled chicken egg after the absorption amount is measured.

(Ovomucoid-Free Shelled Chicken Egg in Calculation Step)

The ovomucoid-free shelled chicken egg in the calculation step is used to derive the above-described conversion formula. The ovomucoid-free shelled chicken egg is as described above. Examples of the ovomucoid-free shelled chicken egg in the calculation step include shelled chicken eggs obtained from chicken species in which the ovomucoid gene is deleted (knocked out) by genetic manipulation or the like. The ovomucoid-free shelled chicken egg in the calculation step may be known to be an ovomucoid-free shelled chicken egg when the absorption amount of the light having the specific wavelength is measured, or may be found to be an ovomucoid-free shelled chicken egg after the absorption amount is measured.

(Method of Calculating Discriminant Value)

The discriminant value is calculated by substituting the absorption amount measured in the measurement step into the conversion formula.

(Selection Step)

The method for selecting ovomucoid-free shelled chicken eggs includes a selection step of selecting an ovomucoid-free shelled chicken egg on the basis of the discriminant value.

(Selection Method)

The selection of ovomucoid-free shelled chicken eggs is performed on the basis of the calculated discriminant

value of the shelled chicken egg. For example, the selection may be performed by comparing the calculated discriminant value of the shelled chicken egg with a preset threshold. The threshold described above may be preset according to the purpose, and for example, from the viewpoint of determination accuracy of ovomucoid-free shelled chicken eggs, the threshold may be set low in order to reduce false positives, and from the economic viewpoint, the threshold may be set high in order to reduce false negatives. That is, the threshold described above can be appropriately set in consideration of how much the false negative rate and the false positive rate are balanced, and the like.

In a case where the conversion formula is derived by PLS-DA from the result of measuring the absorption amount with the discriminant value of the ovomucoid-containing shelled chicken egg being 1 and the discriminant value of the ovomucoid-free shelled chicken egg being 0, for example, the shelled chicken egg may be selected as an ovomucoid-free shelled chicken egg in a case where the discriminant value of the shelled chicken egg for which the absorption amount is measured is less than the threshold. The threshold may be, for example, within a range of more than 0 and 0.6 or less, may be within a range of 0.05 or more and 0.6 or less, 0.1 or more and 0.6 or less, 0.1 or more and 0.5 or less, 0.1 or more and 0.4 or less, or 0.1 or more and 0.3 or less, or may be within a range of 0.2 or more and 0.6 or less, 0.3 or more and 0.5 or less, or 0.4 or more and 0.5 or less. Furthermore, since the proportion of ovomucoid-free shelled chicken eggs in the selected shelled chicken eggs increases as the threshold is set to be lower, in a case where it is desired to reduce false positives, the threshold may be set within a range of, for example, 0.1 or more and 0.4 or less, 0.1 or more and 0.35 or less, 0.1 or more and 0.3 or less, 0.1 or more and 0.25 or less, or 0.1 or more and 0.2 or less. Since the ratio at which ovomucoid-free shelled chicken eggs are selected increases as the threshold is set to be higher, in a case where it is desired to reduce false negatives, the threshold may be set within a range of, for example, 0.35 or more and 0.6 or less, 0.4 or more and 0.6 or less, 0.45 or more and 0.6 or less, or 0.5 or more and 0.6 or less. In a case where it is desired to reduce false negatives while reducing false positives, the threshold may be set within a range of, for example, 0.2 or more and 0.5 or less, 0.2 or more and 0.4 or less, 0.2 or more and 0.35 or less, 0.3 or more and 0.5 or less, 0.35 or more and 0.5 or less, 0.4 or more and 0.5 or less, or 0.3 or more and 0.4 or less.

In the method for selecting ovomucoid-free shelled chicken eggs according to the present embodiment, the measurement step to the selection step may be performed once, and then a part or all of the measurement step to the selection step may be further performed a plurality of times. In this case, it may be performed under conditions different from those in the measurement step to the selection step at the first time. For example, measuring of the absorbance by changing the wavelength of light having a specific wavelength in the measurement step, performing the selection step while changing the above-described threshold in the selection step, and the like are mentioned.

(Method for Producing Ovomucoid-Free Shelled Chicken Egg)

The present invention is characterized by providing a method for producing ovomucoid-free shelled chicken eggs, the producing method including a step of selecting an ovomucoid-free shelled chicken egg by performing the above-described method for selecting ovomucoid-free shelled chicken eggs.

The method for selecting ovomucoid-free shelled chicken eggs is as described above.

## Examples

Hereinafter, the present invention will be more specifically described based on Examples and the like. However, the present invention is not limited to the following Examples.

[Test Example 1: Determination (1) of Ovomucoid-Free Shelled Chicken Egg]

(1. Derivation of Conversion Formula)

A total of 453 ovomucoid-containing shelled chicken eggs and ovomucoid-free shelled chicken eggs (both eggs in a non-destructive state) for conversion formula derivation shown in the following Table 1 were irradiated with light having a wavelength of 310 nm or more and 1100 nm or less, and absorbance (absorption spectrum) of the light having the specific wavelength was measured. Thereafter, the conversion formula was derived using measurement data of absorbance.

The absorbance (absorption spectrum) of the light having the specific wavelength was measured by detecting transmitted light by emitting light having a wavelength of 310 nm or more and 1100 nm or less in a direction intersecting a major axis connecting a rounded end and a pointed end of the shelled chicken egg for conversion formula derivation in a state where the major axis was disposed to be substantially perpendicular to the horizontal plane. The measurement data of absorbance was acquired using a visible near infrared spectrophotometer. The measuring device and conditions for the absorbance (absorption spectrum) are as follows.

Device: Handy Lambda II NIR enh (manufactured by Spectra Co-op.)

Analysis software: The Unscrambler X (manufactured by CAMO Software)

The conversion formula was derived by the following procedure. The discriminant value set such that the discriminant value of the ovomucoid-containing shelled chicken egg for conversion formula derivation was 1 and the discriminant value of the ovomucoid-free shelled chicken egg for conversion formula derivation was 0 was used as the dependent variable. Savitzky-Golay fitting was performed on the measured absorption spectrum using 25 points on each side for smoothing , and second-order differentiation (The Unscrambler X (manufactured by CAMO Software)) was performed. PLS-DA was performed on the dependent variable with the second derivative value of the absorbance of the light having the specific wavelength in the absorption spectrum after the second-order differentiation as an explanatory variable. The $R^2$ determination coefficient of the derived conversion formula was a high numerical value of 0.579, and the goodness of fit of the conversion formula was high.

(2. Determination of Ovomucoid-Free Shelled Chicken Egg)

A total of 125 ovomucoid-containing shelled chicken eggs and ovomucoid-free shelled chicken eggs (both eggs in a non-destructive state) for evaluation shown in the following Table 1 were similarly irradiated with light having a wavelength of 310 nm or more and 1100 nm or less. Next, the absorbance (absorption spectrum) of the light having the specific wavelength was measured, a discriminant value was calculated using the conversion formula derived in the above 1., and then the discriminant value was compared with a threshold to determine whether or not the chicken egg is an ovomucoid-free shelled chicken egg.

The absorption spectrum was measured in the same manner as in the above 1. Thereafter, the discriminant value was calculated by substituting the absorbance of light having the same wavelength as when the conversion formula was derived in the measured absorption spectrum of the shelled chicken egg into the conversion formula. For the selection of ovomucoid-free shelled chicken eggs, the thresholds were set to 0.5, 0.4, 0.3, 0.2, and 0.1, and in a case where the discriminant values were less than the respective thresholds, the shelled chicken eggs were determined as ovomucoid-free shelled chicken eggs. Table 2 shows the results of evaluating Determination Rate 1 calculated by the following Formula (1), and Table 3 shows the results of evaluating Determination Rate 2 calculated by the following Formula (2).

Discrimination Rate 1 (%) = (Number of correct answers of ovomucoid-free shelled chicken eggs/ Number of shelled chicken eggs having discriminant value less than threshold) × 100 Formula (1)

Discrimination Rate 2 (%) = {(Number of correct answers of ovomucoid-free shelled chicken eggs + Number of correct answers of ovomucoid-containing shelled chicken eggs)/Total number of evaluated shelled chicken eggs} × 100 Formula (2)

[Table 1]

| Sample | For conversion formula derivation | For evaluation |
|---|---|---|
| Ovomucoid-containing shelled chicken eggs obtained from wild-type chicken species (number) | 157 | 43 |
| Ovomucoid-free shelled chicken eggs obtained from ovomucoid knocked-out chicken species 1 (number) | 177 | 46 |
| Ovomucoid-free shelled chicken eggs obtained from ovomucoid knocked-out chicken species 2 (number) | 119 | 36 |
| Total (number) | 453 | 125 |

[Table 2]

| Case of setting threshold to 0.5 | |
|---|---|
| Discrimination rate 1 | 96.30% |
| Number of correct answers of ovomucoid-free shelled chicken eggs (number) | 78 |
| Number of shelled chicken eggs having discriminant value less than threshold (number) | 81 |

Case of setting threshold to 0.4

| Discrimination rate 1 | 97.47% |
|---|---|
| Number of correct answers of ovomucoid-free shelled chicken eggs (number) | 77 |
| Number of shelled chicken eggs having discriminant value less than threshold (number) | 79 |

Case of setting threshold to 0.3

| Discrimination rate 1 | 97.06% |
|---|---|
| Number of correct answers of ovomucoid-free shelled chicken eggs (number) | 66 |
| Number of shelled chicken eggs having discriminant value less than threshold (number) | 68 |

Case of setting threshold to 0.2

| Discrimination rate 1 | 100.00% |
|---|---|
| Number of correct answers of ovomucoid-free shelled chicken eggs (number) | 53 |
| Number of shelled chicken eggs having discriminant value less than threshold (number) | 53 |

Case of setting threshold to 0.1

| Discrimination rate 1 | 100.00% |
|---|---|
| Number of correct answers of ovomucoid-free shelled chicken eggs (number) | 44 |
| Number of shelled chicken eggs having discriminant value less than threshold (number) | 44 |

[Table 3]

| Case of setting threshold to 0.5 | |
|---|---|
| Discrimination rate 2 | 94.40% |
| Number of correct answers of ovomucoid-free shelled chicken eggs + number of correct answers of ovomucoid-containing shelled chicken eggs (number) | 118 |
| Total number of evaluated shelled chicken eggs (number) | 125 |

Case of setting threshold to 0.4

| Discrimination rate 2 | 94.40% |
|---|---|
| Number of correct answers of ovomucoid-free shelled chicken eggs + number of correct answers of ovomucoid-containing shelled chicken eggs (number) | 118 |

(continued)

| Total number of evaluated shelled chicken eggs (number) | 125 |
|---|---|

Case of setting threshold to 0.3

| Discrimination rate 2 | 85.60% |
|---|---|
| Number of correct answers of ovomucoid-free shelled chicken eggs + number of correct answers of ovomucoid-containing shelled chicken eggs (number) | 107 |
| Total number of evaluated shelled chicken eggs (number) | 125 |

Case of setting threshold to 0.2

| Discrimination rate 2 | 76.80% |
|---|---|
| Number of correct answers of ovomucoid-free shelled chicken eggs + number of correct answers of ovomucoid-containing shelled chicken eggs (number) | 96 |
| Total number of evaluated shelled chicken eggs (number) | 125 |

Case of setting threshold to 0.1

| Discrimination rate 2 | 69.60% |
|---|---|
| Number of correct answers of ovomucoid-free shelled chicken eggs + number of correct answers of ovomucoid-containing shelled chicken eggs (number) | 87 |
| Total number of evaluated shelled chicken eggs (number) | 125 |

As shown in Table 2, in a case where the threshold was set to 0.5, Determination Rate 1 was 96.30%. Furthermore, as the threshold was set to a lower value, Determination Rate 1 tended to be higher. From this point, it is considered that this is because the number of ovomucoid-containing shelled chicken eggs determined as ovomucoid-free shelled chicken eggs (false positives) is reduced. From this result, it was shown that ovomucoid-free shelled chicken eggs can be selected with high accuracy by the method performed in Test Example 1.

Further, as shown in Table 3, Determination Rate 2 calculated by the calculation method as in Formula (2) tended to be lower as the threshold was set to a lower value. This is considered to be because, as the threshold is lowered, the number of false positives decreases, while the number of ovomucoid-free shelled chicken eggs determined as ovomucoid-containing shelled chicken eggs (false negatives) is increased. Conversely, as the threshold was set to a higher value, Determination Rate 2 tended to be higher. This is considered to be because the number of ovomucoid-free shelled chicken eggs, which were false-negative, was reduced. Therefore, from the results of Tables 2 and 3, it was shown that ovomucoid-free shelled chicken eggs can be selected with high accuracy by appropriately setting a threshold according to the purpose.

[Test Example 2: Determination (2) of Ovomucoid-Free Shelled Chicken Egg]

(1. Derivation of Conversion Formula)

The conversion formula was derived in the same manner as in Test Example 1 except that the wavelength of light with which the shelled chicken egg was irradiated was changed and the wavelength of light having the specific wavelength was changed accordingly. A total of 119 ovomucoid-containing shelled chicken eggs and ovomucoid-free shelled chicken

eggs (both eggs in a non-destructive state) for conversion formula derivation shown in the following Table 4 were irradiated with light having a wavelength of 800 nm or more and 2500 nm or less, and absorbance (absorption spectrum) of the light having the specific wavelength was measured. Thereafter, the conversion formula was derived using measurement data of absorbance. The $R^2$ determination coefficient of the derived conversion formula was a considerably high numerical value of 0.701, and the degree of fitness of the conversion formula was considerably high.

(2. Determination of Ovomucoid-Free Shelled Chicken Egg)

The ovomucoid-free shelled chicken egg was determined in the same manner as in Test Example 1 except that the wavelength of light with which the shelled chicken egg was irradiated was changed and the wavelength of light having the specific wavelength was changed accordingly. A total of 29 ovomucoid-containing shelled chicken eggs and ovomucoid-free shelled chicken eggs (both eggs in a non-destructive state) for evaluation shown in the following Table 4 were similarly irradiated with light having a wavelength of 800 nm or more and 2500 nm or less. Next, the absorbance (absorption spectrum) of the light having the specific wavelength was measured, a discriminant value was calculated using the conversion formula derived in the above 1. of Test Example 2, and then the discriminant value was compared with a threshold to determine whether or not the chicken egg is an ovomucoid-free shelled chicken egg. Table 5 shows the results of evaluating Determination Rate 1 calculated by the above Formula (1), and Table 6 shows the results of evaluating Determination Rate 2 calculated by the above Formula (2).

[Table 4]

| Sample | For conversion formula derivation | For evaluation |
|---|---|---|
| Ovomucoid-containing shelled chicken eggs obtained from wild-type chicken species (number) | 59 | 15 |
| Ovomucoid-free shelled chicken eggs obtained from ovomucoid knocked-out chicken species 1 (number) | 60 | 14 |
| Total (number) | 119 | 29 |

[Table 5]

| Threshold | Number chicken of eggs shelled having discriminant value less than threshold (number) | Number of correct answers of ovomucoid- free shelled chicken eggs (number) | Discrimination rate 1 (%) |
|---|---|---|---|
| 0.5 | 18 | 13 | 72.2 |
| 0.4 | 16 | 11 | 68.8 |
| 0.3 | 11 | 8 | 72.7 |
| 0.2 | 10 | 7 | 70.0 |
| 0.1 | 8 | 6 | 75.0 |

[Table 6]

| Threshold | Total number of evaluated shelled chicken eggs (number) | Number of correct answers of ovomucoid-free shelled chicken eggs + number of correct answers of ovomucoid-containing shelled chicken eggs (number) | Discrimination rate 2 (%) |
|---|---|---|---|
| 0.5 | 29 | 23 | 79.31 |
| 0.4 | 29 | 21 | 72.41 |
| 0.3 | 29 | 20 | 68.97 |
| 0.2 | 29 | 19 | 65.52 |
| 0.1 | 29 | 19 | 65.52 |

Even when the wavelength of the light having the specific wavelength was changed, the same results as in Test Example 1 were obtained. As the threshold was set to a lower value, Determination Rate 1 tended to be higher and Determination Rate 2 tended to be lower. Since the conversion formula derived in Test Example 2 has a higher $R^2$ determination coefficient than the conversion formula derived in Test Example 1, it is considered that the determination accuracy becomes higher by further increasing the number of lots. From the above result, it was shown that ovomucoid-free shelled chicken eggs can be selected with high accuracy also by the method performed in Test Example 2.

## Claims

1. A method for selecting shelled chicken eggs, the method being a selecting method for ovomucoid-free shelled chicken eggs, comprising:

   a measurement step of irradiating a shelled chicken egg with light including light having a specific wavelength to measure an absorption amount of the light having the specific wavelength;
   a calculation step of calculating a discriminant value by substituting the absorption amount into a conversion formula; and
   a selection step of selecting an ovomucoid-free shelled chicken egg on the basis of the discriminant value, wherein
   the conversion formula is a formula derived from a result of measuring the absorption amount of the light having the specific wavelength for an ovomucoid-containing shelled chicken egg and an ovomucoid-free shelled chicken egg.

2. The selecting method according to claim 1, wherein the light having the specific wavelength is light existing within a wavelength range of 310 nm or more and 2500 nm or less.

3. The selecting method according to claim 1 or 2, wherein the conversion formula is a formula derived by partial least squares-discriminant analysis.

4. The selecting method according to claim 1 or 2, wherein the conversion formula is a formula derived by partial least squares-discriminant analysis with a discriminant value of the ovomucoid-containing shelled chicken egg as 1 and a discriminant value of the ovomucoid-free shelled chicken egg as 0,

   in the selection step, in a case where the discriminant value calculated in the calculation step is less than a threshold, the egg is selected as an ovomucoid-free shelled chicken egg, and
   the threshold is within a range of more than 0 and 0.6 or less.

5. A method for producing ovomucoid-free shelled chicken eggs, the producing method comprising:
   a step of selecting an ovomucoid-free shelled chicken egg by performing the selecting method according to claim 1 or 2.

## INTERNATIONAL SEARCH REPORT

International application No. **PCT/JP2024/038918**

**A. CLASSIFICATION OF SUBJECT MATTER**

***G01N 21/27***(2006.01)i; ***G01N 33/08***(2006.01)i
FI: G01N21/27 Z; G01N21/27 F; G01N33/08

According to International Patent Classification (IPC) or to both national classification and IPC

**B. FIELDS SEARCHED**

Minimum documentation searched (classification system followed by classification symbols)

G01N21/00-G01N21/61,G01N33/00-G01N33/98

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII)

**C. DOCUMENTS CONSIDERED TO BE RELEVANT**

| Category* | Citation of document, with indication, where appropriate, of the relevant passages | Relevant to claim No. |
|---|---|---|
| A | JP 6686216 B1 (KEWPIE CORPORATION) 22 April 2020 (2020-04-22) | 1-5 |
| A | JP 2006-36669 A (KEWPIE CORPORATION) 09 February 2006 (2006-02-09) | 1-5 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **25 December 2024** | **14 January 2025** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**
**Information on patent family members**

International application No.
**PCT/JP2024/038918**

| Patent document cited in search report | Publication date (day/month/year) | Patent family member(s) | Publication date (day/month/year) |
|---|---|---|---|
| JP 6686216 B1 | 22 April 2020 | WO 2020/111262 A1 | |
| JP 2006-36669 A | 09 February 2006 | (Family: none) | |

Form PCT/ISA/210 (patent family annex) (July 2022)

## REFERENCES CITED IN THE DESCRIPTION

*This list of references cited by the applicant is for the reader’s convenience only. It does not form part of the European patent document. Even though great care has been taken in compiling the references, errors or omissions cannot be excluded and the EPO disclaims all liability in this regard.*

### Patent documents cited in the description

- JP 2017049177 A
- JP 5959061 B